Europäisches Patentamt

European Patent Office

Office européen des brevets

(11) Publication number: 0 376 135 A2

# EUROPEAN PATENT APPLICATION

(21) Application number: 89123508.7

(22) Date of filing: 20.12.89

(51) Int. Cl.5: G01N 33/52

(30) Priority: 29.12.88 US 291846

(43) Date of publication of application:
04.07.90 Bulletin 90/27

(84) Designated Contracting States:
DE FR GB IT NL

(71) Applicant: W.R. Grace & Co.-Conn. (a
Connecticut corp.)
Grace Plaza 1114 Avenue of the Americas
New York New York 10036(US)

(72) Inventor: Parham, Marc Ellous
9 Ruben Duren Way
Bedford MA 01730(US)
Inventor: Raina, Santosh
8 Hazel Road
Lexington MA 02173(US)
Inventor: King, Donald Perry, Jr.
2 1/2 Fifteenth Avenue
Haverhill MA 01830(US)
Inventor: Blinn, Diane C.
475 Rollstone Street
Fitchburg MA 01420(US)

(74) Representative: UEXKÜLL & STOLBERG
Patentanwälte
Beselerstrasse 4
D-2000 Hamburg 52(DE)

(54) Improved dipstick device for assays.

(57) A dipstick is disclosed which can be used in assays to determine the presence or absence of a target analyte in a viscous liquid or a liquid which comprises cellular or particulate matter. The dipstick is a thermoplastic support strip to which is welded a wide-pore woven or non-woven material which has been precoated with protein nonadsorptive polyurethane. A bioaffinity agent is immobilized on the wide-pore material.

FIG. I

## IMPROVED DIPSTICK DEVICE FOR ASSAYS

### BACKGROUND OF THE INVENTION

This invention relates generally to assay devices. More specifically, a fabric-based dipstick device has been developed which can be used to detect analytes in fluids which are viscous, colloidal suspensions or which contain cellular and/or particulate materials. Most particularly, the dipstick device of this invention is useful for detecting various compounds which may be present in milk, such as progesterone or antibiotics, for example. Due to the design of this novel device, the target compound or analyte in the test sample has enhanced access to bioaffinity agents immobilized on a porous fabric support for rapid binding thereto, notwithstanding interference due to the described conditions (i.e., viscosity of the test sample, interference from cellular or particulate matter, etc.).

Diagnostic assays most frequently are conducted on microporous membrane supports, such as nylon, which conventionally are prepared in the following manner. A solution containing a bioaffinity agent is dotted onto the membrane and dried, becoming immobilized on the membrane by passive adsorption or through the use of binding agents. Next, at least one blocking step is required for the prevention of nonspecific binding of proteins which may be present in the test sample or reagent solutions. Nonspecific binding across the entire membrane renders the assay inaccurate and unreadable.

The assay typically is conducted by passing a test fluid through the membrane. If the target compound is present in the test fluid, it will bind to a unique binding site of the bioaffinity agent (e.g., primary antibody) immobilized on the membrane. The membrane then is treated with a recognition conjugate, consisting of a recognition antibody (which binds to a second binding site on the target compound) coupled to a detection compound. For colorimetric assays, the detection compound is an enzyme, such as horseradish peroxidase, which can be induced to generate a color change under positive test conditions. The membrane is rinsed and treated with a substrate for the enzyme-mediated color change reaction. If the target compound was present in the test fluid, it will have become bound to the primary antibody and, in turn, will have bound the recognition conjugate. The enzyme portion of the recognition conjugate will react with the substrate, producing an easily detected color change for a positive assay. If the target compound was not present, the recognition conjugate will not be bound to the membranes and no membrane color change will occur upon treatment with the substrate.

Dipstick devices are widely known and used for certain diagnostic assays. The conventional dipstick designs are similar to those disclosed in U.S. 4,125,372 (Kawai et al.) or U.S. 4,308,028 (Elkins), in which reference bodies or reagent pads are immobilized on a strip which is neutral to the test sample. The device is contacted with a test liquid such as urine. In this type of assay, the non-viscous test liquid is absorbed by the reagent pad, allowing for good contact between the pad and the test liquid (including the target compound, if present).

A microfiltration membrane-based dipstick device is disclosed in European Patent Application No. 87101071.6 (Incstar Corporation) for use in solid phase enzyme immunoassays and nucleic acid hybridization assays in which the test fluid is human blood serum. Various cellulosic materials and activated polyamide membrane in pore sizes of about 0.2-10.0 microns are suggested for use as the membrane support. The membrane is held rigidly in a dipstick of planar design.

Neither conventional dipstick designs (absorbent pads adhered to a strip) nor the Incstar dipstick design (rigidly held microporous membrane in a planar configuration) are particularly suitable for use with test samples which are viscous, colloidal suspensions (such as milk) or which contain cellular and/or particulate materials (such as whole blood). Assays conducted under these conditions are not amenable to the conventional dipstick format since insufficient absorption occurs for good contact between the test sample and the bioaffinity agent immobilized in or on the absorbent pad. Reduced contact due to viscosity or particulate interference of the fluid sample will result in greatly reduced sensitivity of the assay, making it difficult to distinguish positive from negative test results.

Nor is the Incstar dipstick design suitable for use under the conditions described here. The microporous membrane used will not permit adequate flow of the test fluid to achieve sufficient contact with the bioaffinity agent. Moreover, the activated nylon membrane surface is not suitable for immobilization of antibodies in a manner resulting in a device with long term stable biological activity.

Instead, tests have been used in these situations such as that disclosed in U.S. 4,716,109 (Baker et al.). Baker et al. teaches a bovine pregnancy test (assaying for the presence of progesterone in milk) in which reagents are added to and mixed with milk sample, which is then observed for the presence or absence of clotting in order to determine the onset of estrus. Similarly, U.S. 4,568,637 (Klein) discloses a method of detecting beta lactam ring-containing cephalosporins and penicillin in biological liquids such as milk. The liquid sample is contacted with a beta lactam ring-containing chromogenic compound and penicillinase, and then color development in the milk itself is measured.

## SUMMARY OF THE INVENTION

The dipstick design of the present invention can be used for rapid assays in viscous fluids or in fluids containing cellular or particulate materials where contact between the test sample and the assay support may be impaired or reduced. The novel dipstick comprises an elongated support strip or holder to which is fixed a porous material. In a preferred embodiment, the strip has one or more apertures near one end which are completely or partially covered by a porous material securely affixed to the holder by ultrasonic welding. In another preferred embodiment, the porous material is securely affixed to the strip in such a manner that a space is present between the porous material and the surface of the strip. The porous material is a wide-pore woven or nonwoven fabric to which is immobilized a bioaffinity agent for use in detecting the target compound or analyte.

It is a primary object of this invention to provide a rapid and convenient dipstick-type assay for use in testing fluids which are viscous or which may contain substantial quantities of cellular or particulate material. It is particularly intended that the invention will provide sensitive dipstick assays for testing milk, cell suspensions or cultures, whole blood, and the like. It is further intended that the dipstick provide for sharp and clear distinctions between positive and negative assay results.

It is a specific object to provide a rapid and convenient device for assessing progesterone levels in the milk of lactating mammals, in order to determine the onset of estrus and to detect pregnancy on a date appropriate relative to a prior insemination attempt.

It is another specific object to provide a rapid and convenient device for detecting antibiotics, such as penicillin, which may be present in the milk of lactating mammals, and for assessing the levels of such antibiotics.

A related object of the invention is to provide a device for an assay which can be conducted quickly and accurately. That is, it is intended to provide an assay device designed for rapid binding of the target compound to affinity sites immobilized on the membrane as well as rapid binding of the recognition conjugate and substrate. Moreover, it is intended that the device design will allow for rapid and thorough rinsing of the dipstick to remove unbound (and potentially interfering) proteins and other materials prior to addition of test reagents.

An additional object is to provide a dipstick assay in an embodiment which has one or more built-in control indicators for rapid identification of results and/or assay failure.

## BRIEF DESCRIPTION OF THE DRAWINGS

FIG. 1 is a front view of one embodiment of the dipstick of this invention in which a single test area is used.

FIG. 2 is a back view of the dipstick of FIG. 1.

FIG. 3 is a front view of a second embodiment of the invention, having a test area and one control area.

FIG. 4 is a back view of the dipstick of FIG. 3 where spearate pieces of porous material are used.

FIG. 5 is a back view of the dipstick of FIG. 3 where a single piece of porous material is used.

FIG. 6 is a front view of a third embodiment of the invention, having a test area and two control areas in a linear configuration.

FIG. 7 is a back view of the dipstick of FIG. 6 where separate pieces of porous material are used.

FIG. 8 is a back view of the dipstick of FIG. 6 where a single piece of porous material is used.

FIG. 9 is a sectional view of the dipstick of FIG. 6, taken along line 9-9.

FIG. 10 is an exploded view of a fourth embodiment of the invention, having a test area and two control areas in a linear configuration, in a sandwich-type dipstick.

FIG. 11 is a sectional view of the dipstick of FIG. 10, taken along line 11-11.

FIG. 12 is a front view of a fifth embodiment of the invention, having a test area and two control areas in a triangular configuration.

FIG. 13 is a front view of a sixth embodiment of the invention, having a test area and two control areas in a triangular configuration.

FIG. 14 is a perspective view of a seventh embodiment of the invention, having a curved piece of porous material affixed to the dipstick.

FIG. 15A is a sectional view of the dipstick of FIG. 14, taken along line 15-15.

FIG. 15B is a sectional view of the dipstick of FIG. 14, taken along line 15-15, showing an alternative embodiment having an aperture through the dipstick portion.

FIG. 16 is a sectional view of an eighth embodiment of this invention, taken through the assay portion of the dipstick, having spacers between the support strip and the porous material.

## DETAILED DESCRIPTION OF THE INVENTION

A dipstick device has been developed which can be used for rapid immunoassay of viscous fluids or fluids containing cellular or particulate material. This device consists of an elongated support strip or holder, near one end of which is affixed a wide-pore woven or nonwoven fabric material. In the preferred embodiment, the device comprises a flexible strip or holder having one or more apertures near one end, over which is affixed a wide-pore material on which bioaffinity reagents are immobilized. This design allows for rapid contact and binding between the immobilized reagent(s) on the dipstick and the test fluid, recognition conjugate and substrate. The design also allows for rapid and convenient rinsing and assay development.

The support strip or holder of which the dipstick is comprised may be made of any material which is insoluble in the test fluid. The material preferably will be stiff, but somewhat flexible, although a rigid material may be used if desired. Most preferably, a plastic such as polypropylene, polystyrene, polyvinyl chloride, high density linear polyethylene, acrylic butadiene styrene copolymers, or other polymeric materials which can be easily ultrasonically welded.

The support strip conveniently is white in color, although other colors, or even colorless material, can be used. It may be preferred to use a color which is intermediate between the colors of positive and negative assay results. For example, blue may be used where a positive result yields white or very pale blue (which is lighter than the support strip color) and a negative result yields dark blue (which is darker than the support strip color). In this way, the support strip itself serves as a comparison for evaluating the results.

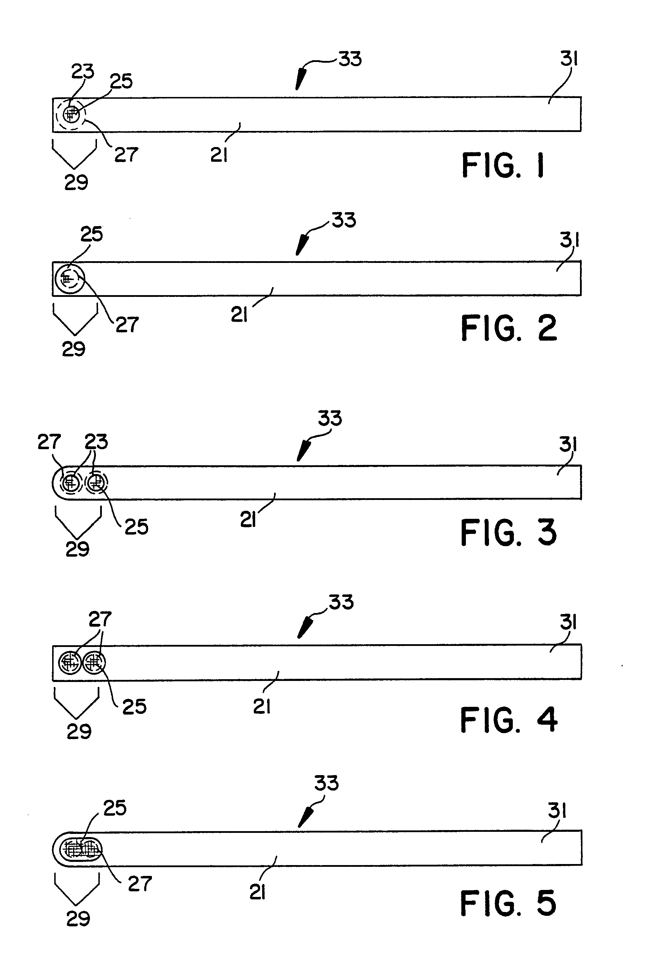

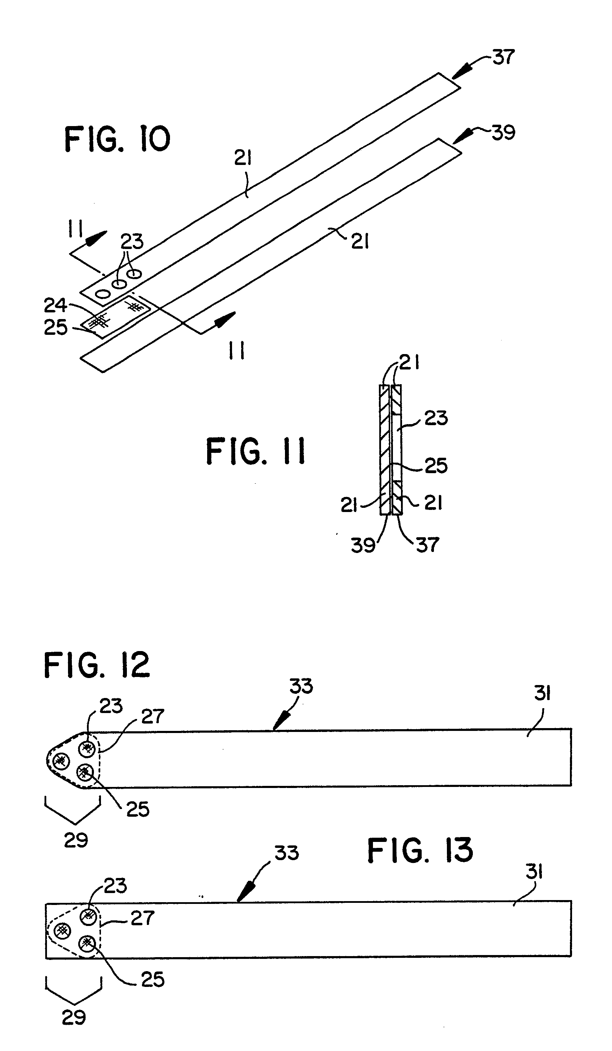

Any dipstick-style holder of convenient size and shape may be used. Most conveniently, an elongated strip on the order of about 1.0 cm by about 10.0-15.0 cm, with a thickness of about 0.03 to 0.2 cm is used. With reference to the figures, support strip 21 may be somewhat varied as to shape and width. Support strip 21 should be of sufficient length to allow assay portion 29 to be inserted into the test sample and reagent solution(s) while handling via distal portion 31. The width of support strip 29 should accommodate a sufficient area of porous material for conduct of the assay and, if desired, one or more controls. This area is depicted in the figures as assay portion 29. The linear configuration depicted in FIGS. 1-8 can be accommodated on a narrow support strip 21, while the triangular configuration depicted in FIGS. 12 and 13 requires a somewhat wider support strip 21. The assay portion also could be placed horizontally across the width of the support strip.

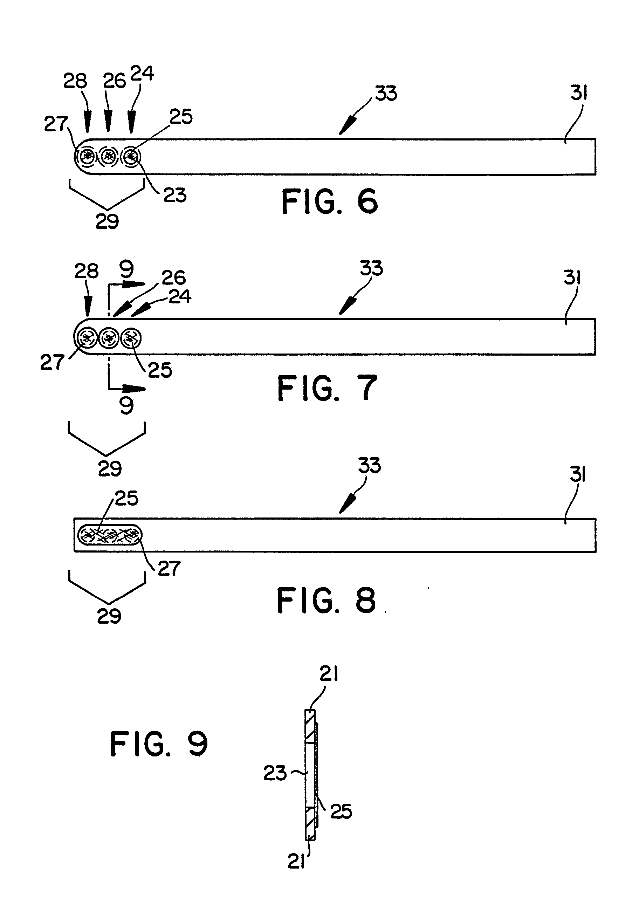

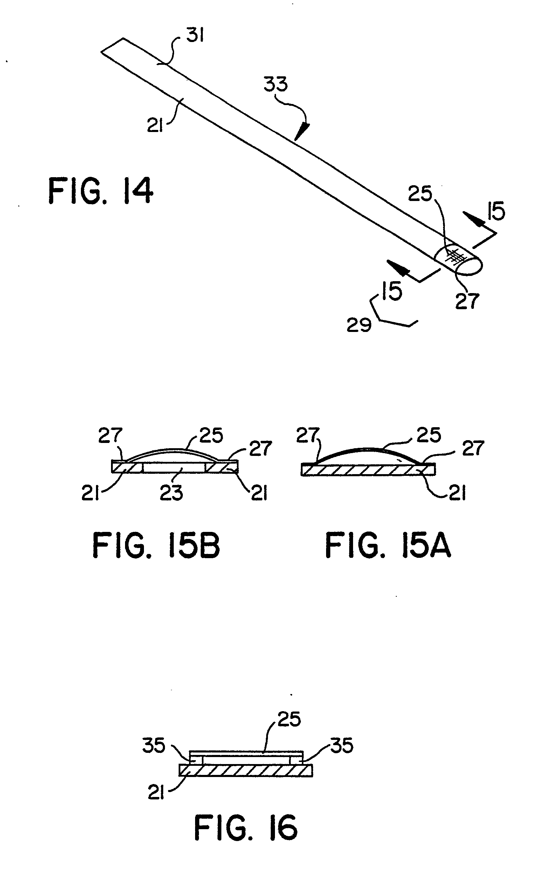

The thickness of support strip 21 should afford it sufficient strength so that it will not tear or break under stress caused, for example, by agitation in the test fluid, rinsing, etc. Once this minimum thickness requirement is met, the strip may be as thick as desired. It can be seen that the desired thickness will vary with the material used for the holder or strip. In addition, the embodiments with apertures 23 through support strip 21, such as shown in FIG. 7, may need to be somewhat thicker or wider to avoid breakage, as compared with an embodiment such as shown in FIG. 14, where support strip 21 remains intact.

In one embodiment of this invention, flexible support strip 21 will have one or more holes or apertures 23, each of which is covered with porous woven or nonwoven material 25 as described below. Porous material 25 provides a support on which the assay is conducted. Apertures 23 are positioned near one end of support strip 21 as shown in FIGS. 1-13. In one variation of this embodiment, there are three holes: one for the assay, one positive control for determining enzyme activity and one negative control for assessing nonspecific protein binding. If more than one such hole 23 is used, the holes may be positioned along the length of support strip 21, as in FIGS. 3-8, or across the width of support strip 21, or in any other desired configuration, such as the triangular configuration shown in FIGS. 12-13.

Apertures 23 must be large enough to accommodate contact of porous material 25 with adequate amounts of test fluid and test reagents to result in a visually apparent result and to achieve desired assay sensitivity. For example, circular apertures with diameters of about 3.0 to about 7.0 mm will be suitable, although the apertures may be of any convenient size and shape relative to the dimensions of the flexible strip.

It is preferred, but not required, that holes or apertures 23 are completely covered with porous material 25. Porous material 25 may be any wide pore woven or nonwoven fabric material which is insoluble in the test fluid and reagents. Porous material 25 also should have sufficient strength characteristics to avoid tearing or separating during the test procedures.

Porous material 25 most preferably is nylon-based. That is, a nonwoven or woven nylon fabric is chosen and then is polymer-treated as described herein to create the porous support material used for the dipstick of this invention. Nylon materials are available having different porosities, densities and thicknesses. Particularly suitable is Cerex™ nonwoven nylon fabric (James River Corp.). Alternatively, porous support material 25 may be wide-pore polypropylene, polyurethane, polysulfones, polyacrylates, various polyesters, polyvinyl fluoride, polyvinyl chloride, or cellulosic materials.

Woven or nonwoven materials may be used. Materials with nominal pore size of at least about 10.0 microns, up to about 30.0 microns may be used. This is designated as a nominal pore size since nonwoven materials typically are not classified by pore size. The interfiber spaces vary in size. However, the distribution of pore sizes should be such that substantial numbers of pores or spaces are at least about 10.0 microns.

The porous material preferably is provided with a protein non-adsorptive polyurethane polymer precoating in order to reduce or eliminate nonspecific binding of protein onto the assay surface and to increase the amount of bioaffinity agent which can be immobilized on the surface. This precoating eliminates the need for separate blocking steps after immobilization of the bioaffinity agent(s). In addition, the urethane coating allows antibodies and other bioaffinity agents to be stably immobilized on the porous support. The result is good retention of antibody or other bioaffinity agent, with the product having a suitably long shelf life. It is preferred that the precoated porous material be aged for at least about seven days, preferably at least about ten days, prior to immobilization of the bioaffinity agent.

The polyurethane polymer used for this precoating preferably is one formed by polymerization of a prepolymer prepared by capping a polyoxyalkylene polyol with a polyisocyanate compound, such that the prepolymer has a reaction functionality greater than two. Particularly preferred is HYPOL™ 6100 polyurethane prepolymer (Grace Specialty Chemicals Co., W. R. Grace & Co.-Conn.). A solution is prepared comprising the polyurethane prepolymer in a volatile organic solvent, (for example, acetone, alcohols, chlorinated hydrocarbons, etc.) in a concentration of about 0.1 to about 20.0 percent prepolymer. A surfactant also may be present. The porous support material is contacted with the solution and then dried. Detailed procedures for coating membranes in this fashion are given in USSN 911,944 (Parham et al.), filed September 26, 1986, which is incorporated herein by reference.

Alternatively, the porous material may be coated with a solution of an aqueous-based polyurethane polymer in a volatile organic solvent, thus forming a polyurethane polymer coating as described in USSN 235,411 (Rudolph et al.), filed August 22, 1988, which is incorporated herein by reference. The material is contacted with a solution comprising an aqueous-based polyurethane polymer and a volatile organic solvent for example, acetone, alcohols, chlorinated hydrocarbons, etc.) in a concentration of about 0.1 to about 20.0 percent polymer. A non-ionic surfactant also may be present. The porous support material is contacted with the solution and then dried. The polymer may be any of the elastomers described in U.S. 4,442,259 (Isgur et al.). Most preferably, the coating is prepared by treatment with Darathane™ WB-22 polyurethane elastomer (Grace Specialty Chemicals Co., W. R. Grace & Co.-Conn.), which is an aliphatic urethane polymer in an aqueous solution of about 40% solids in ethanol. A dual polymer coating comprising Darathane elastomer and a second polyurethane polymer may be used. For example, a solution of Darathane WB-22 elastomer (4%) and HYPOL 6100 prepolymer (1%) in acetone may be used to coat the porous material.

Although it is preferred to pre-coat the porous support material as described above, the untreated material can be used, if desired. This embodiment would be best suited for applications which are relatively protein-free, for example, with urine-based assays. However, it has been found that acceptable results are obtained in milk-based assays, although color development may be less intense due to milk proteins displacing antibodies on the assay surface.

The polymer-coated porous support material is further pre-treated by immobilization of one or more bioaffinity agents. The bioaffinity agent(s) can be affixed to the porous material at this stage or after the material is attached to the support strip of the dipstick. As shown in the figures, at least one test area 24 comprising porous support material 25 is present in assay portion 29 of dipstick 33. Relatively large quantities of the polymer-coated porous material can be pretreated with appropriate bioaffinity agents prior to affixing the material to the support strip. Alternatively, the bioaffinity agent(s) may be applied to the porous material after dipstick 33 has been assembled. The appropriate reagent solutions may be dotted onto porous material 25 after it has been affixed to assay portion 29 of support strip 21. The efficacy of assembled dipstick 33 is not expected to vary with the order of the preparation steps.

The dipstick device of this invention is useful in any of a variety, of diagnostic or other assays and the specific bioaffinity agents will be determined by the assay. The bioaffinity agent can be any reagent capable of reacting with, or otherwise detecting, a target analyte in the test sample. It is applied to the porous material using known immobilization techniques. The bioaffinity agent (e.g., a selected antibody, either polyclonal or monoclonal, a protein or antigen, etc.) conveniently may be applied directly to the porous material and dried. Alternatively, the active reagent together with a binding aid (e.g., hydroxyethylcellulose) may be deposited on the porous material and dried. These immobilization techniques are well known to those of ordinary skill in the art.

The bioaffinity agent is immobilized on a first polymer-coated porous support by applying the agent and drying. FIGS. 1-2 demonstrate the use of a single test area 24 on which the agent is fixed. In alternative embodiments, two or more separate areas of assay portion 29 may be employed, as shown in FIGS. 4-8. For example, a second porous support, otherwise identical to the first, may be treated with the recognition agent used in the assay (e.g., antibody to the enzyme used) and will serve as a control to detect the presence of active recognition agent (enzyme). A third porous support, otherwise identical to the first and second, which is left blank with respect to active agents, can serve as a control to detect nonspecific binding of proteins. Other test areas may be included, as additional controls or for added tests.

FIGS. 3-5 demonstrate the use of test area 24 and either negative control 26 or positive control 28. Where a single control area is used, it preferably is a negative control for detection of nonspecific protein binding. It may be preferred to omit the enzyme control described above, due to cross-reaction between the enzyme control area and the test area. FIGS. 6-13 show embodiments utilizing test area 24 and both negative control 26 and positive control 28 in assay portion 29 of dipstick 33. In addition, the embodiment shown in FIG. 14 could be constructed to incorporate test area 24 and negative control 26 by dotting the bioaffinity agent in one portion (i.e., the center) of porous material 25. In this way, a positive test will depend on the presence or absence of a colored dot, whereas failure of the negative control will result in an overall color change over the whole of porous material 25.

A piece or pieces of pretreated porous materials 25 are affixed to support strip 21 so that each hole 23 is partially or wholly covered with porous support material 25. In the embodiment utilizing three pre-treated porous materials 25 (test surface, positive enzyme control, negative nonspecific binding control), appropriately sized pieces of the three polymer-coated and pre-treated support materials are affixed over the three apertures 23, one per aperture. Dipsticks with one or two apertures are similarly constructed.

Ultrasonic welding is the preferred method for fixing or attaching porous material 25 to support strip 21. The attachment means must be sufficient to securely fasten the membrane to the holder so that it will not be removed or loosened either while in contact with the test liquid or during assay or rinsing procedures. Preferably, dipstick 33 should be able to withstand forceful rinsing, as under a faucet or other stream of water. Creation of ultrasonic welding seam 27 accomplishes these goals without interfering with the assay. Ultrasonic welding of the materials used for the dipstick of this invention is accomplished without the use of solvents, heat or adhesives. This method also gives excellent adhesion of porous material 25 to support strip 21 along ultrasonic welding seam 27 with no adverse effect on either porous material 25 or bioaffinity agents with which that material may have been pretreated.

Ultrasonic welding is appropriate where the two materials (here, the support strip and porous material) have relatively close melting points, preferably within about 20°C. The ultrasonic process provides very localized vibration which heats, and melts, the thermo plastic material of support strip 21 and/or porous material 25, welding them together under pressure to form seam 27. Since the effect of the ultrasonic vibration is so localized, the assay portions of the porous support material are not damaged or altered and bioaffinity agents which may have been immobilized on the material are not denatured. Ultrasonic welding thus provides a benign method for securely fastening porous material 25 to support strip 21 in order to fashion dipstick 33 of this invention. A wide variety of thermoplastic polymers can be welded in this manner.

In the embodiment depicted in FIGS. 6-8, the completely assembled dipstick device 33 has three porous material-covered holes or apertures 23 near one end (generally forming assay portion 29). The first such covered hole, test area 24, has primary antibodies or other bioaffinity agents immobilized on the porous material. The second covered hole, negative control area 26 for detection of nonspecific protein binding, has no immobilized reagents on the porous material. The third covered hole, positive control area 28 for enzyme activity, has secondary antibodies (or other agents reactive with the assay recognition enzyme or recognition agent) immobilized on the porous material. Colorimetric results are read by holding assay portion 29 of dipstick 33 flat against any white background. Particularly where positive control area 28 is used, it is most preferred for ultrasonic welding seam to form a continuous seal around each aperture 23 to reduce "cross-talk" between the separate areas of assay portion 29.

In another embodiment, one or more pieces of porous material 25 can be sandwiched between two support strips 21 as shown in FIGS. 10 and 11. This configuration requires the presence of one or more holes or apertures 23 in at least first strip 37, in order for the test fluid to have access to porous material 25. In one variation, only first strip 37 has one or more holes. Second strip 39 acts as the white background against which the results are read. This is demonstrated in an exploded view in FIG. 10. In another variation, both first strip 37 and second strip 39 have corresponding holes 23 which are aligned when porous material 25 is sandwiched between support strips 21. In this manner, the test fluid contacts the porous material on both exposed surfaces. It is preferred that both first strip 37 and second strip 39 have holes 23 corresponding to the location of test area 24. Once the two strips and porous material are properly positioned in the sandwich configuration, they may be ultrasonically welded to form the dipstick 33.

An alternative embodiment, depicted in FIGS. 14 and 15A, utilizes support strip 21 in which no holes or apertures 23 are made. A piece of porous material 25, preferably square or rectangular, is affixed to assay portion 29 of support strip 21 by ultrasonic welding in the following manner. Porous material 25 is welded to the strip along two opposite, substantially parallel sides or edges creating ultrasonic welding seams 27; the remaining two sides or edges of porous material 25 are left unattached to the strip. Ultrasonic welding seams 27 of porous material 25 are positioned on strip 21 such that the porous material bends or projects outwardly from the strip in an arc along a midline of the material, as shown in FIG. 15A. This projection allows for greater contact between porous material 25 and the test sample, as does the flexibility imparted to the porous material by virtue of this design. In addition, this design aids in reading the assay by spacing porous material 25 from support strip 21. Using an embodiment in which support strip 21 is white and no holes are present allows for instantaneous reading of the assay results, without the need for a separate white surface against which the assay results may be read.

In still another alternative embodiment, related to that shown in FIG. 14, one or more holes or apertures 23 may be made in that portion of support strip 21 which is located between ultrasonic welding seams 27, as shown in FIG. 15B. The hole(s) 23 will further increase flow and contact between the test fluid and the porous material 25, and will aid in rinsing the porous material.

In another embodiment, a piece of porous material is affixed to spacers 35 which are, in turn, affixed to support strip 21 as shown in FIG. 16. To allow fluid access to both surfaces of porous material 25, support strip 21 may have one or more apertures 23 in assay portion 29. Alternatively, the spacers 35 may be present along two opposing edges of porous material 25. This configuration also will allow rapid fluid access to both surfaces of porous material 25 for efficient test fluid and reagent interaction as well as efficient washing. Where no holes or apertures 23 are present in the strip, the dipstick 33 contains its own white background against which colorimetric results are easily readible.

The assay is conducted by contacting assay portion 29 of the dipstick with the test fluid and with appropriate test reagents. The dipstick may be held (either manually or mechanically) at distal portion 31. A small sample of the test fluid is taken for use in the assay. In one preferred embodiment (an enzyme-conjugate colorimetric assay), the enzyme-conjugate is added to the test fluid and mixed thoroughly. Assay portion 29 of the dipstick is placed into the test fluid/enzyme-conjugate mixture so that porous material 25 is completely immersed. The dipstick is incubated in the test sample for at least about one to five minutes, preferably about two minutes, at room temperature. The necessary incubation period will depend on the reagents used, the degree of agitation and the desired sensitivity of the particular assay being conducted.

Agitation of the dipstick in the test sample is preferred to enhance contact between the test area and the analyte to be detected. It has been found that agitation decreases the time necessary for the assay, presumably due to the overall increased analyte-porous material contact. In addition, where the assay is conducted in a viscous fluid, such as milk, more consistant results are achieved with agitation. Agitation may be done manually or mechanically, and may be by motion of the dipstick or the test sample, or both.

It is preferred to agitate the test sample mechanically by placing a vial or other container on or in a shaker mechanism. The vial or container preferably is at a slight angle from upright, i.e., about a 10° to 30° angle, but may be at any convenient angle of about 5°-70°. Agitation in this manner has been found to reduce the necessary dipstick-test fluid contact time by about one-half. Upright agitation is acceptable, but is not preferred. The most appropriate agitation speed, angle and time period can easily be determined for each assay to be conducted using the dipstick of this invention. As an example, a Tek-Tator V™ agitator (American Dade) may be used at a speed of about 220 rpm for about two minutes.

The dipstick is removed from the test sample and rinsed thoroughly, preferably by holding assay portion 29 under a stream of cold running water for about 20 seconds. Excess water then is removed, as by blotting with an absorbent material such as a paper towel or pad. The dipstick is incubated in a solution containing a developing substrate for the enzyme and is again blotted to remove excess liquid.

The dipstick is then examined to determine whether the target compound or analyte was present in the test sample. This may be done visually or using a reflectometer or other automated means. In colorimetric assays, color changes in the test area of the porous material will indicate the test result. In embodiments utilizing a positive enzyme activity control, the color of the positive control area will indicate activity of the enzyme. In embodiments utilizing a negative nonspecific binding control, the negative control area should remain unchanged (e.g., white) or may be very slightly tinged with color, relative to an external color standard comparison chart, indicating little or no nonspecific binding of enzyme or other reactive protein.

In one preferred assay to be used with the dipstick of this invention, a first porous support material (i.e., the test area) is treated with an antibody to progesterone. A progesterone assay is described here for explanatory purposes only. This invention is not limited to use with progesterone assays, but can be used for other purposes, as described below.

The progesterone assay described here is based on a colorimetric assay, in which the recognition conjugate consists of a recognition antibody for progesterone and an enzyme such as horseradish peroxidase, alkaline phosphatase, or glucose oxidase which cause a visually apparent color change under positive test conditions and in the presence of a substrate specific for the enzyme. A substrate used with horseradish peroxidase is tetramethyl benzidine (TMB), with or without hydrogen peroxide. Substrates used with alkaline phosphatase are indoxyl phosphate, with or without nitro blue tetrazoleum (NBT). A substrate used with glucose oxidase is 2,2′-azino-di-(3-ethylbenzthiazoline-G-sulfonic acid) (ABTS). With certain enzymes (e.g., alkaline phosphatase), the color change appears on the porous material of the dipstick. With other enzymes (e.g., horseradish peroxidase), the color change appears in the test solution itself. Colorimetric assays also can be based on colloidal gold, rather than enzymes.

A second preferred assay to be used with the dipstick of this invention tests for the presence of the antibiotic penicillin. For example, cow's milk must be tested to determine penicillin levels in order to be sold for human consumption. For penicillin testing, penicillin binding proteins (the bioaffinity agent) are immobilized on the porous material used on the dipstick. In a colorimetric assay embodiment, the dipstick is added to a solution of milk and a penicillin-enzyme conjugate. The results are indicated by the presence or absence of a color change.

Other bioaffinity agents may be selected for other assays. For example, luteinizing hormone (LH), human chorionic gonadotropin (HCG) and other hormones, steroids such as estrone or progesterone, antibiotics such as penicillin, infectious disease agents such as group A strep, Chlamydia, gonorrhea, syphilis, herpes, Candida, Trichomonas, HIV, and the like also may be detected with assay devices prepared according to this invention. Antigens, RNA or DNA probes and the like, can be used in place of immobilized antibody.

Nor is the utility of the dipstick devices of this invention limited to colorimetric assays. These dipsticks may be used with any convenient recognition and tracer or indicator system. For example, the recognition antibody may be radiolabeled for use in a radioimmunoassay. Alternatively, the indicator can be a fluorescent labelling agent that is bound to an antibody or bioaffinity agent. Examples include fluorescein isocyanate (FIC), fluoroscein isothiocyanate (FITC), 7-amino-4-methylcoumarin-3-acetic acid (AMCA) and the like. These and other adaptations and applications are within the knowledge and ability of those of ordinary skill in the art. It can be seen, however, that the objects of speed and simplicity of the invention are best achieved when the dipstick is used in conjunction with a simple enzyme-based colorimetric detection system.

For some assays, quantitative determinations also may be made. That is, the assay may be designed so that the intensity of color change will correspond to the presence of low to high levels of the target analyte in the test sample. Color development in the test area of the dipstick may be compared with a standardized color chart indicating levels of color intensity and the corresponding target analyte concentrations. Comparisons may be made visually or with automated equipment, such as a reflectometer.

In another method for conducting assays with the dipstick of this invention, the test reagents are serially applied to the dipstick test surface. The dipstick is dipped into the test fluid so that the porous support material is completely immersed in the test liquid. Preferably, the dipstick is used to agitate the fluid somewhat or the test container is shaken, in order to increase fluid flow through the porous material, thereby increasing contact between the immobilized bioaffinity agent and the target analyte, if present in the fluid. The dipstick then may be allowed to remain in contact with the test sample for up to about 30 seconds or more.

The dipstick is removed from the test sample, thoroughly rinsed and then incubated with the appropriate detection reagents. For example, the dipstick may be incubated sequentially in appropriate recognition-conjugate and developing substrates, preferably with rinsing between. The negative control area, if present, should remain unchanged. The positive enzyme activity control area, if present, should develop a detectable signal. The test area will indicate positive or negative results as described above.

The time periods required for incubation in the test sample and developing reagents will depend in part on the nature and quality of the reagents. The color change, both in terms of color and intensity, also will depend on the reagent system used. Similarly, where other detection systems are used (i.e., fluorescence), the intensity of the signal will depend in part on the system and reagents used. The advantage of the dipstick device of this invention is in the rapid saturation of binding sites on the immobilized bioaffinity agent, notwithstanding viscosity of the sample or intereference by cellular or particulate material.

The examples which follow are given for illustrative purposes and are not meant to limit the invention described herein. The following abbreviations have been used throughout in describing the invention:

cm - centimeter(s)
cm² - square centimeter(s)
mg - milligram(s)
ml - milliliter(s)
mm - millimeter(s)
μg - microgram(s)

## EXAMPLE I

### (Preparation of Dipstick Device)

Plastic polystyrene strips were cut as shown in FIG. 6, with three holes near one end of each strip. The strips were approximately 13.0 cm x 1.0 cm. The holes were approximately 4.0 mm in diameter.

Cerex™ nonwoven nylon fabric (James River Corp.) was treated with a 1% solution of HYPOL™ 6100 polyurethane prepolymer (Grace Specialty Chemicals Co., W. R. Grace & Co.-Conn.) in acetone containing 0.1% Tween-20™ (ICI United States, Inc.) for sixty minutes. The fabric was removed from the solution, allowed to drip dry in air and then was air dried at room temperature for 18 hours.

A piece of the dried fabric of appropriate size was ultrasonically welded to each polystyrene strip so as to just cover all three holes. Next, 0.5 μg murine monoclonal antibody to progesterone (Immuno-search, Inc.) was immobilized by drying onto the fabric over one hole. An antibody against alkaline phosphatase (the enzyme used in the assay) was immobilized on that portion of the fabric covering a second hole in order to provide a positive control for the enzyme. The fabric over the third hole was left blank to serve as a control to detect nonspecific binding of milk proteins and as a control to ensure that complete rinsing was accomplished.

The treated dipsticks were dried overnight, then soaked in 0.1 M Tris buffer (pH 7.4) for one hour. The buffer was discarded, the dipsticks washed once with fresh buffer and allowed to dry.

## EXAMPLE II

### (Dipstick Assay)

The dipsticks prepared in Example I were used in an ELISA assay using alkaline phosphatase as the enzyme. For each assay, enzyme-conjugate (progesterone linked to alkaline phosphatase (Immuno-search, Inc.)) containing 0.07 units of enzyme activity was added to 0.6 ml cow's milk and mixed thoroughly. One dipstick was incubated in each sample for about five minutes at room temperature.

The dipsticks were removed and washed thoroughly under a stream of cold running water for about five seconds. Excess water was removed by blotting with an absorbent material. The dipsticks were then each incubated for about three minutes in a separate solution of enzyme substrate, indoxyl phosphate (Hybritech) sufficient to cover all three holes. Indoxyl phosphate produces a dark blue color (indigo) in the presence of the enzyme alkyline phosphatase. The dipsticks were removed from the substrate solution and blotted to absorb excess solution. The porous support covering the three holes was visually observed to detect any color change. The color was compared to a standardized color chart to determine whether each milk sample contained high, low or no levels of progesterone. In negative milk samples, or in samples containing low Levels of progesterone, the assay portion turned blue in color. In samples containing high levels of progesterone, the assay portion was white or very pale blue, that is, a lower intensity of color than the reference standard color chart used for comparison.

## EXAMPLE III

(Dipstick Assay)

The dipsticks prepared in Example I were used in an ELISA assay using horseradish peroxidase as the enzyme. For each assay, enzyme-conjugate (progesterone linked to horseradish peroxidase) containing 0.002 units of enzyme activity was added to 0.6 ml cow's milk and mixed thoroughly. One dipstick was incubated in each sample with manual agitation for about two minutes at room temperature.

The dipsticks were removed and washed thoroughly under a stream of cold running water for about five seconds. Excess water was removed by blotting with an absorbent material. The dipsticks were then each incubated for about three minutes in a separate solution of enzyme substrate, tetramethyl benzidine. The color was compared to a standardized color chart to determine whether each milk sample contained high or low levels of progesterone. In samples containing low levels of progesterone, the enzyme substrate solution turned blue in color. In samples containing high levels of progesterone, the enzyme substrate solution was white or very pale blue, that is, a lower intensity of color than the reference standard color chart used for comparison.

## EXAMPLE IV

(Preparation of Dipstick Device)

Plastic polystyrene strips were cut as shown in FIG. 6, with three holes near one end of each strip, although only two holes were used in this example. The strips were approximately 13.0 cm x 1.0 cm. The holes were approximately 4.0 mm in diameter.

Cerex™ nonwoven nylon fabric (James River Corp.) was treated with a 1% solution of HYPOL™ 6100 polyurethane prepolymer (Grace Specialty Chemicals Co., W. R. Grace & Co.-Conn.) in acetone containing 0.1% Tween-20™ (ICI United States, Inc.) for sixty minutes. The fabric was removed from the solution, allowed to drip dry in air and then was air dried at room temperature for 18 hours.

A solution containing 1.0 mg/ml penicillin binding proteins in phosphate buffered saline (PBS) was applied to the prepolymer-treated fabric by saturating the fabric with the solution and then drying overnight, in order to immobilize the protein onto the fabric. A piece of the dried fabric of appropriate size was ultrasonically welded to each polystyrene strip so as to just cover one hole. A piece of prepolymer-treated fabric (without immobilized protein) was ultrasonically welded over one of the remaining holes on each polystyrene strip.

The dipsticks were dried overnight, then soaked in 0.1 M Tris buffer (pH 7.4) for one hour. The buffer was discarded, the dipsticks washed once with fresh buffer and allowed to dry.

## EXAMPLE V

(Dipstick Assay)

The dipsticks prepared in Example IV were used in a penicillin ELISA assay using alkaline phosphatase as the enzyme. For each assay, enzyme-conjugate (penicillin linked to alkaline phosphatase) was added to 1.0 ml cow's milk and mixed thoroughly. One dipstick was incubated in each sample for about five minutes at room temperature with no agitation.

The dipsticks were removed and washed thoroughly with cold water. The dipsticks were then incubated in a substrate solution (indoxyl phosphate) for three minutes to develop the color. In negative milk samples, the fabric portion of the dipstick turned dark blue. In positive milk samples (i.e., containing penicillin), the fabric remained white or turned a very pale blue.

## EXAMPLE VI

(Preparation of Dipstick Device)

Plastic polystyrene strips were cut as shown in FIG. 1 with one hole near one end of each strip. The strips were approximately 13.0 cm x 1.0 cm. The holes were approximately 4.0 mm in diameter.

Cerex ™ nonwoven nylon fabric (James River Corp.) was treated with a 1% solution of HYPOL™ 6100 polyurethane prepolymer (Grace Specialty Chemicals Co., W. R. Grace & Co.-Conn.) in acetone for sixty minutes. The fabric was removed from the solution, allowed to drip dry in air and then was air dried at room temperature for ten days.

A solution containing monoclonal antibody to beta-human chorionic gonadotropin ($\beta$-HCG) (with a binding constant of $10^{10}$) in phosphate buffered saline (PBS) was applied to the prepolymer-treated fabric by saturating the fabric with the solution and then drying overnight, in order to immobilize the antibody onto the fabric. Unbound antibody was washed off in 0.1 M Tris buffer (pH 7.5). A piece of the fabric of appropriate size was ultrasonically welded to each polystyrene strip so as to just cover the hole.

### EXAMPLE VII

The dipsticks prepared in Example VI were used in a competition assay to detect the presence of human chorionic gonadotropin ($\beta$-HCG) using alkaline phosphatase as the enzyme. For each assay, one dipstick was incubated for about three minutes with agitation in urine containing enzyme-conjugate ($\beta$-HCG linked to alkaline phosphatase).

The dipsticks were removed and washed thoroughly with cold water. The dipsticks were then incubated in a substrate solution (indoxyl phosphate) for three minutes to develop the color. In this competition assay, if the urine sample contains $\beta$-HCG, no enzyme-conjugate will bind to the antibody. Therefore, the assay portion of the dipstick turned blue where the urine sample was negative for $\beta$-HCG and stayed white where the urine sample was positive for $\beta$-HCG.

The principles, preferred embodiments and modes of operation of the present invention have been described in the foregoing specification. The invention which is intended to be protected herein, however, is not to be construed as limited to the particular forms disclosed, since these are to be regarded as illustrative rather than restrictive. Variations and changes may be made by those skilled in the art without departing from the spirit of the invention.

## Claims

1. A dipstick for assaying a viscous liquid or a liquid which comprises cellular or particulate matter in order to detect the presence of a target analyte in said liquid, said dipstick comprising:

   (a) a thermoplastic support strip,

   (b) one or more pieces of wide-pore woven or non-woven material having a protein nonadsorptive polyurethane coating thereon, and

   (c) one or more bioaffinity agents, wherein said wide-pore material is affixed by ultrasonic welding in a position adjacent to one end of said support strip in such a manner that upon insertion of said dipstick into said liquid, the liquid has access to both surfaces of said wide-pore material, and wherein at least one bioaffinity agent is immobilized on said wide-pore material.

2. The dipstick of Claim 1 in which said support strip has one or more holes adjacent to one end, with one or more pieces of said wide-pore material ultrasonically welded about the perimeter of said hole or holes in such a manner that said hole or holes are at least partially covered by said wide-pore material.

3. The dipstick of Claims 1 or 2 in which said support has:

   (i) test area which is a first hole about which is welded wide-pore material having a bioaffinity agent immobilized thereon, and

   (ii) a positive control area which is a second hole about the perimeter of which is welded a wide-pore material having immobilized thereon a secondary agent reactive with the recognition agent used in the assay.

4. The dipstick of Claims 1 to 3 in which said support has:

   (i) a test area which is a first hole about which is welded wide-pore material having a bioaffinity agent immobilized thereon,

   (ii) a positive control area which is a second hole about which is welded wide-pore material having immobilized thereon a secondary agent reactive with the recognition agent used in the assay, and

   (iii) a negative control area which is a third hole about which is welded wide-pore material.

5. The dipstick of Claims 1 to 4 in which said wide-pore material comprises pores with nominal pore size of at least about 10.0 microns.

6. The dipstick of Claims 1 to 5 in which said bioaffinity agent comprises antibodies, antigens, proteins, RNA probes or DNA probes.

7. The dipstick of Claims 1 to 6 in which said wide-pore material projects outwardly from said support strip along a midline of said material, and said wide-pore material is ultrasonically welded to said strip along two edges which are substantially parallel to each other and to said midline.

8. The dipstick of Claims 1 to 7 in which spacers are arranged between said wide-pore material and said support strip along two substantially parallel edges of said wide-pore material such that said wide-pore material is elevated from said strip to create a path for fluid access to both surfaces of said wide-pore material.

9. A method for directly assaying a viscous liquid or a liquid which comprises cellular or particulate matter in order to detect the presence in said liquid of a target analyte by contacting a dipstick comprising a wide-pore woven or non-woven material having a bioaffinity agent immobilized thereon with a test sample of said liquid, a recognition agent-assay conjugate and developing substrate for said recognition agent, wherein said dipstick comprises:

(a) a thermoplastic support strip,

(b) one or more pieces of wide-pore woven or non-woven material having a protein nonadsorptive polyurethane coating thereon, and

(c) one or more bioaffinity agents, wherein said wide-pore material is affixed by ultrasonic welding in a position adjacent to one end of said support strip in such a manner that upon insertion of said dipstick into said liquid, the liquid has access to both surfaces of said wide-pore material, and wherein at least one bioaffinity agent is immobilized on said wide-pore material.

10. The method of Claim 9 in which positive or negative assay results are determined by detecting the presence or absence of color changes on the test area of said dipstick.

11. The method of Claim 9 in which positive or negative assay results are determined by detecting the presence or absence of color change in the test sample.

12. The method of Claim 9 which comprises:

(a) mixing said recognition agent-assay conjugate with said test sample,

(b) incubating said dipstick in said mixture,

(c) removing and rinsing said dipstick,

(d) incubating said dipstick in developing substrate,

(e) removing and rinsing said dipstick, and

(f) reading the assay results by determining the presence or absence of a color change.

13. The method of Claim 12 which comprises manually or mechanically agitating the mixture of test sample and recognition agent-assay conjugate during the incubation of step (b).

14. The method of Claims 9 to 13 in which the container holding said mixture is at an angle of between about 5° and 70° from upright.

15. The method of claims 9 to 14 which comprises

(a) incubating said dipstick in said test sample,

(b) removing and rinsing said dipstick,

(c) incubating said dipstick in recognition agent-assay conjugate solution,

(d) removing and rinsing said dipstick,

(e) incubating said dipstick in developing substrate,

(f) removing and rinsing said dipstick, and

(g) reading the assay results by determining the presence or absence of a color change.

16. The method of Claim 15 which comprises manually or mechanically agitating the test sample during the incubation of step (a).

17. The method of Claims 14 to 16 in which the container holding said test sample is at an angle of between about 5° and 70°C from upright.

18. A dipstick for assaying a viscous liquid or a liquid which comprises cellular or particulate matter in order to detect the presence of a target analyte in said liquid, said dipstick comprising:

(a) a thermoplastic support strip,

(b) one or more pieces of wide-pore woven or non-woven material, and

(c) one or more bioaffinity agents, wherein said wide-pore material is affixed by ultrasonic welding in a position adjacent to one end of said support strip in such a manner that upon insertion of said dipstick into said liquid, the liquid has access to both surfaces of said wide-pore material, and wherein at least one bioaffinity agent is immobilized on said wide-pore material.

FIG. 1

FIG. 2

FIG. 3

FIG. 4

FIG. 5

FIG. 6

FIG. 7

FIG. 8

FIG. 9

FIG. 10

FIG. 11

FIG. 12

FIG. 13

FIG. 14

FIG. 15B

FIG. 15A

FIG. 16